# EUROPEAN PATENT SPECIFICATION

(11) **EP 1 435 794 B1**
(45) Date of publication and mention of the grant of the patent: **17.11.2010**
(21) Application number: 02758285.7
(22) Date of filing: 28.06.2002
(51) Int. Cl.: A23K 1/14, A23K 1/16, A23K 1/18

(54) **PET FOOD COMPOSITION FOR REGULATING BODY WEIGHT AND PREVENTING OBESITY AND RELATED DISORDERS IN PETS**
HAUSTIERFUTTERZUSAMMENSETZUNG ZUR REGULIERUNG DES KÖRPERGEWICHTS UND ZUR VERHINDERUNG DER FETTLEIBIGKEIT UND ÄHNLICHER STÖRUNGEN BEI HAUSTIEREN
COMPOSITION D'ALIMENT POUR ANIMAUX PERMETTANT DE REGULER LE POIDS VIF ET DE PREVENIR L'OBESITE AINSI QUE DES TROUBLES ASSOCIES CHEZ LES ANIMAUX

(30) Priority: 17.07.2001 EP 01202719
(43) Date of publication of application: 14.07.2004
(73) Proprietor: Société des Produits Nestlé S.A., 1800 Vevey (CH)
(72) Inventor: LEMAURE, Bernard, F-37000 Tours (FR); DARIMONT-NICOLAU, Christian, CH-1005 Lausanne (CH)
(74) Representative: Lock, Graham James
(86) International application number: PCT/EP2002/007208
(87) International publication number: WO 2003/007732

(56) References cited:
- EP-A- 0 631 727
- WO-A-01/03691
- WO-A-01/17364
- WO-A-01/21008
- JP-A- 6 133 731
- US-A- 5 962 043
- US-B1- 6 245 364
- DATABASE WPI Section Ch, Week 199209 Derwent Publications Ltd., London, GB; Class B04, AN 1992-068562 XP002187281 & JP 04 011865 A (MEIJI SEITO), 16 January 1992 (1992-01-16)
- DATABASE WPI Section Ch, Week 200014 Derwent Publications Ltd., London, GB; Class B04, AN 2000-156621 XP002187282 & JP 2000 016916 A (KANEBO LTD), 18 January 2000 (2000-01-18)
- N. SINGH ET AL.: "A pharmacological study of Cyperus rotundus" INDIAN JOURNAL OF MEDICAL RESEARCH., vol. 58, no. 1, 1970, pages 103-109, XP001042333 INDIAN COUNCIL OF MEDICAL RESEARCH, NEW DEHLI., IN ISSN: 0019-5340
- DATABASE WPI Section Ch, Week 200174 Derwent Publications Ltd., London, GB; Class B04, AN 2001-642013 XP002220994 & JP 2001 226274 A (MATSUURA YAKUGAKU KK), 21 August 2001 (2001-08-21)
- H. MEYER ET AL.: "Ein Beitrag zur Vorkommen und zur Behandlung der Adipositas des Hundes" DEUTSCHE TIERÄRZTLICHE WOCHENSCHRIFT, vol. 85, no. 4, 1978, pages 133-136, XP001058151 DE
- M.YOSHIOKA, ET AL.: "Effects of Capsaicin on abdominal fat and serum free-fatty acids in exercise-trained rats" NUTRITIONAL RESEARCH, vol. 20, no. 7, 2000, pages 1041-1045,
- E.L. HOVE ET AL.: "The effect of lucerne-protein concentrate in the diet on growth, reproduction and body composition of rats" BRITISH JOURNAL OF NUTRITION, vol. 31, 1974, page 147-157, XP009123981

## Description

This invention relates to a pet food composition for regulating body weight and preventing obesity and related disorders in pets. The invention also relates to a method for improving the health of pets, particularly by preventing obesity and metabolic disorders associated with obesity in pets. It also relates to a method for increasing energy expenditure, control the glucose homeostasis and improve activity or mobility of the pet.

### Background of the Invention

Obesity is caused by insufficient exercise or habitual hyperphagia, or by metabolic disturbance due to genetic causes or endocrine diseases and other. Obesity may be a risk factor that causes various diseases.

Therefore, early therapeutic and preventive treatment of obesity is very important. Diet therapies or exercise therapies have been applied heretofore as the treatment of mild obesity, and drug therapies are sometimes used for serious obesity in combination with these therapies.

WO 01/03691 describes a composition and method for promoting weight loss in a mammal by feeding an effective amount of at least three nutrients selected from L-carnitine, chromium, Vitamin A and a source of carbohydrates selected from a low glycemic index grain.

US 5,962,043 and US B1 6,245,364 describe a food composition containing a small but sufficient amount of simmondsin and its use in weight reduction of companion pets.

H. Meyer et al.: "Ein Beitrag zur Vorkommen und zur Behandlung der Adipositas des Hundes" Deutsche Tierärztliche Wochen-Schrift, vol. 85, no. 4, 1978, pages 133-136, XP001058151 DE describes an investigation into the frequency of obesity in dogs and includes a feeding and digestibility trial using different diets. One of the diets improves physical conditions of a dog, such a body weight, and comprises lucerne (*Medicago sativa*) but does not comprise seeds therefrom.

Database WPI Section Ch, Week 200014 Thomson Scientific, London, GB; Class B04, AN2000-156621 XP002187282 & JP 2000 016916 A (Kanebo Ltd) 18 January 2000 (2000-01-18) describes a lypolysis promoter and skin cosmetic for a lean figure, comprising one or more ingredients selected from extracts of Coleusforskolii, L-carnitine, yucca, and jasmine.

JP06133731A describes a diet food containing forskolin for use in preventing obesity.

M. Yoshioka et al.: "Effects of Capsaicin on abdominal fat and serum free-fatty acids in exercise-trained rats", Nutritional Research, vol. 20, no.7, 2000, pages 1041-1045, discloses that capsaicin obtained from capsicum fruits, such as hot red pepper, has a beneficial effect on body weight (body fat control).

The stimulation by synthetic agonists of the (β3-adrenergic receptor (β3-AR), which is an atypical beta-adrenergic receptor expressed essentially in adipose tissues, has been shown to produce anti-obesity and anti-diabetic effects in rodents and in dogs (Sasaki et al. 1998, J. Vet. Med. Sci. 60 (4): 459-463). In human, β3-AR is expressed at low levels and its pharmacological properties differ from rodents and dogs.

However, there is a need for a non-detrimental and efficient nutritional way of regulating body weight of pets and treating or preventing obesity, weight gain and related disorders.

### Summary of the Invention

Accordingly, in a first aspect, the present invention provides a pet food composition for use in the treatment and/or the prevention of obesity and/ or related disorders, which comprises as an active ingredient at least one plant or plant extract having the ability to stimulate energy expenditure and/or fat burning, wherein the plant or plant extract is selected from seeds of *Medicago saliva,* leaves of *Capsicum* species, leaves or tubers of *Cyperus* species or tubers of *Iris Pallida,* or a mixture thereof.

In another aspect, the invention provides a pet food composition for use in controlling the glucose homeostasis of a pet and/or stimulating lipolysis, which comprises a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof.

In yet another aspect, the invention provides a pet food composition for use in improving activity and/or mobility of a pet, which comprises a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof.

In addition, the invention relates to the use of at least one plant or plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof as an active ingredient in the manufacture of a pet food composition for the treatment and/or prevention of obesity and/or related disorders.

The invention further relates to the use of a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof, in the manufacture of a pet food composition for controlling the glucose homeostasis of a pet and/or stimulating lipolysis.

It also relates to the use of use of a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof, in the manufacture of a pet food composition for improving activity and/or mobility of a pet.

Administering to a pet, a food composition as described above, results in an improved regulation of body weight, thus treating or preventing obesity and/or related disorders in pets. This food composition increases energy expenditure, lipolysis and lipid oxidation and consequently reduces the mass of white adipose tissue and the total body weight. Furthermore the increase in glucose utilisation improves the glucose homeostasis.

### Detailed Description of the Invention

The plant or plant extract according to the invention has been selected for its ability to stimulate energy expenditure and/or fat burning in adipose tissue of pets. In particular, it may be selected for its ability to stimulate the adrenergic receptors.

In a preferred embodiment, the said plant or plant extract may contain a natural source of β3-adrenergic receptor agonist.

The plant or plant extract is selected from seeds of *Medicago sativa,* leaves of *Capsicum* species, leaves or tubers of *Cypemes* species, tubers of Iris *Pallida* or a mixture thereof.

The plant or plant extract according to the invention may be used in the preparation of a pet food composition. The said composition may be administered to the pet as a supplement to its normal diet or as a component of a nutritionally complete pet food, and more preferably in an hypocaloric pet food. It may also be a pharmaceutical composition.

The plant or plant extract may be used alone or in association with other bioactive molecule such as antioxidants, milk hydrolysates, for example.

Preferably, the pet food composition contains about 0.03 to 0.3 g of dry plants per gram of dry pet food for a 15 kg dog; and 0.007 to 0.075 g of dry plants per gram of wet pet food for a 15 kg dog.

The nutritionally complete pet food composition according to the invention may be in powdered, dried form or a wet, chilled or shelf stable pet food product. These pet foods may be produced by ways known in the art. Apart from the plant or plant extract, these pet foods may include any one or more of a starch source, a protein source and a lipid source.

Suitable starch sources are, for example, grains and legumes such as corn, rice, wheat, barley, oats, soy, and mixtures of these.

Suitable protein sources may be selected from any suitable animal or vegetable protein source; for example meat and meal, poultry meal, fish meal, soy protein concentrates, milk proteins, gluten, and the like. For elderly animals, it is preferred for the protein source to contain a high quality protein.

Suitable lipid sources include meats, animal fats and vegetable fats.

The choice of the starch, protein and lipid sources will be largely determined by the nutritional needs of the animal, palatability considerations, and the type of product applied. For elderly pets, the pet food preferably contains proportionally less fat than pet foods for younger pets. Furthermore, the starch sources may include one or more of rice, barley, wheat and corn.

The pet food may optionally also contain a prebiotic, a probiotic micro-organism or another active agent, for example a long chain fatty acid. The amount of prebiotic in the pet food is preferably less than 10% by weight. For example, the probiotic may comprise about 0.1% to about 5% by weight of the pet food. For pet foods which use chicory as the source of the prebiotic, the chicory may be included to comprise about 0.5% to about 10% by weight of the feed mixture; more preferably about 1% to about 5% by weight.

If a probiotic micro-organism is used, the pet food preferably contains about 10⁴ to about 10¹⁰ cells of the probiotic micro-organism per gram of the pet food; more preferably about 10⁶ to about 10⁸ cells of the probiotic micro-organism per gram. The pet food may contain about 0.5% to about 20% by weight of the mixture of the probiotic micro-organism; preferably about 1% to about 6% by weight; for example about 3% to about 6% by weight.

Suitable long chain fatty acids include linoleic acid, alpha-linolenic acid, gamma linolenic acid, eicosapentanoic acid, and docosahexanoic acid. Fish oils are a suitable source of eicosapentanoic acids and docosahexanoic acid. Borage oil, blackcurrent seed oil and evening primrose oil are suitable sources of gamma linoleic acid. Safflower oils, sunflower oils, corn oils and soybean oils are suitable sources of linoleic acid.

If necessary, the pet food is supplemented with minerals and vitamins so that they are nutritionally complete. Further, various other ingredients, for example, sugar, salt, spices, seasonings, flavouring agents, and the like may also be incorporated into the pet food as desired.

For dried pet food a suitable process is extrusion cooking, although baking and other suitable processes may be used. When extrusion cooked, the dried pet food is usually provided in the form of a kibble. If a prebiotic is used, the prebiotic may be admixed with the other ingredients of the dried pet food prior to processing. A suitable process is described in European patent application No 0850569. If a probiotic micro-organism is used, the organism is preferably coated onto or filled into the dried pet food. A suitable process is described in European patent application No 0862863.

For wet food, the processes described in US patents 4,781,939 and 5,132,137 may be used to produce simulated meat products. Other procedures for producing chunk type products may also be used; for example cooking in a steam oven. Alternatively, loaf type products may be produced by emulsifying a suitable meat material to produce a meat emulsion, adding a suitable gelling agent, and heating the meat emulsion prior to filling into cans or other containers.

The amount of pet food to be consumed by the pet to obtain a beneficial effect will depend on the size of the pet, the type of pet, and age of the pet. However, an amount of the pet food to provide a daily amount of about 0.5 to 5 g of dry plants per kg of body weight, would usually be adequate for dogs and cats.

Administering to a pet, a food composition as described above, results in an improved regulation of body weight, thus treating or preventing obesity and/or related disorders in pets. This composition has a beneficial impact on the stimulation of energy expenditure in pets. It also increases lipolysis and lipid oxidation and consequently reduces the mass of white adipose tissue and the total body weight.

It may also be efficient in the control of the glucose homeostasis, which aims to prevent the development of diabetes, for example. It may further be used to improve stamina, which results in a better activity or mobility of the pet.

The following examples are given by way of illustration only and in no way should be construed as limiting the subject matter of the present application. All percentages are given by weight unless otherwise indicated. The examples are preceded by a brief description of the figures.
**Figure 1** : β3 adrenoreceptor activity (percentage of control) of *Capsicum* species extracts, **A***: Capsicum annuum* var. Sereno (250 µg/ml) and B: *Capsicum frutescens* (250 µg/ml).
**Figure 2** : β3 adrenoreceptor activity of 4 fractions of *Cyperus rotondus* (250 µg/ml), **1**: Hexane extract, **2**: Ethyl acetate extract after HCl hydrolysis, **3**: Methanol/H₂O extract, **4**: as 3 but w/o polyphenols.
**Figure 3** **:** effects of different doses of the hexane extract of *Cyperus rotondus* on β3 adrenoreceptor activity.
**Figure 4****:** β3 adrenoreceptor activity of Medicago Sativa/ Ethyl acetate extract (A) and Iris Pallida/ Hexane extract (B).

### Example 1 : β3 adrenoreceptor activity of Capsicum species extracts

### Material & methods

Four different extracts were prepared as described below from dried Capsicum leaves:
(1) Hexane extract: Lipids, sterols, mono and diterpenes, aliphatic hydrocarbures, aliphatic alcools...
(2) Ethyl acetate extract after HCl hydrolysis: Flavones, flavonols, quinones, organic acids, phenol acids, coumarines, lactones, low subsitued di-tri- and tetra-terpenes, sterols, pigments, alkaloids, lignans...
(3) Methanol/H₂O extract: heterosides from the aglycones present in the extract (2), polyphenols, carbohydrates, hydrosoluble alkaloids, small peptides, amino acids, anthocyanosides....
(4) as (3) but w/o polyphenols. PVPP columns

The β3 adrenoreceptor activity was measured by incubating plant extracts for 1 hour with differentiated rodent preadipocytes previously loaded with [¹⁴C] Acetate. Isoproterenol, a non-specific β adrenoreceptor agonist, was used at 1 µM as a positive control. The culture medium was collected at the end of the incubation and the released-labelled fatty acids were counted with a gamma counter. The cpm counted was normalised with the protein content per well. The values are expressed as percentage of the control value.

### Results.

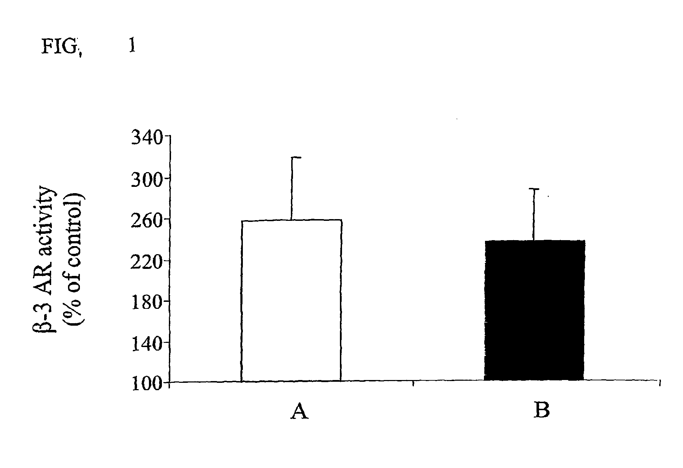

The results are given in figure 1, which shows that the extracts of *Capsicum Sereno* and *Frutescens* have a significant effect on β3-adrenoreceptor activity.

### Example 2: Presence of β3 adrenergic agonists in Cyperus Rotondus and Alfalfa

### Methods

Binding assay was performed on membranes of rat adipose tissue. Membrane were incubated for 90 min at 37°C in the presence of [¹²⁵I] cyanopindolol, a β3 adrenergic agonist, and the plant extracts tested at 250 µg/ml. Following incubation, the membranes were rapidly filtered under vacuum though glass fibre filters. Filters were then washed several times with an ice-cold buffer and bound radioactivity was measured. Results are expressed as a percent inhibition of control values obtained in the presence of plant extracts.

### Results

The extracts of *Cyperus Rotondus* and Medicago Sativa were able to inhibit by and 66% and 44% the binding of [¹²⁵I] cyanopindolol, respectively.

These results clearly show that these plant extracts contain compounds able to bind to the β3 adrenergic receptors.

### Example 3: β3 adrenoreceptor activity of Cyperus Rotondus, Medicago Sativa and Iris Pallida extracts

### Material & methods

Four different extracts were prepared as described in example 1 from Cyperus leaves. The β3 adrenoreceptor activity was measured by incubating plant extracts for 1 hour with differentiated rodent preadipocytes previously loaded with [¹⁴C] Acetate. Isoproterenol, a non-specific β adrenoreceptor, was used at 1 µM as a positive control. The culture medium was collected at the end of the incubation and the released-labelled fatty acids were counted with a gamma counter. The cpm counted was normalised with the protein content per well. The values are expressed as percentage of the control value.

### Results.

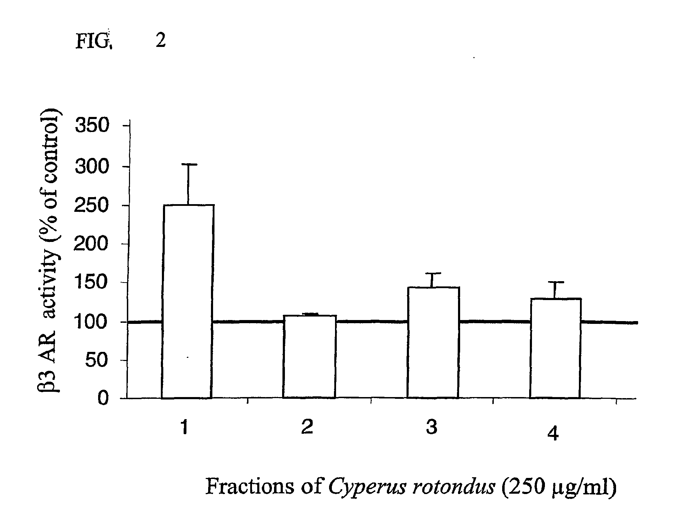

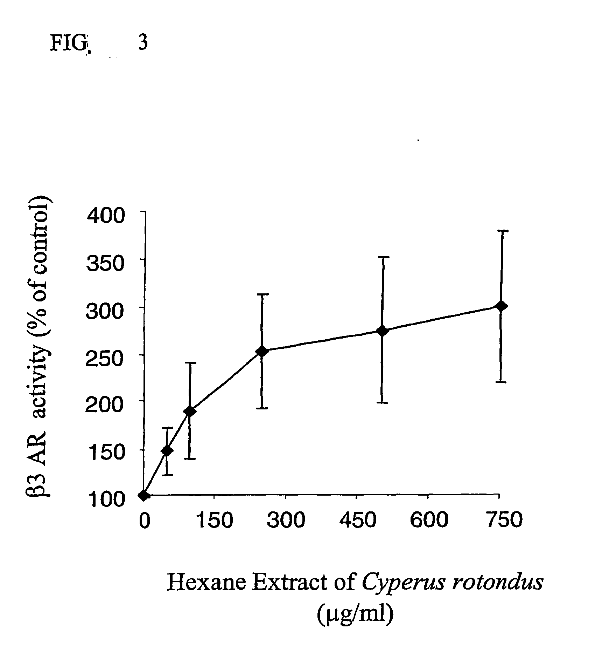

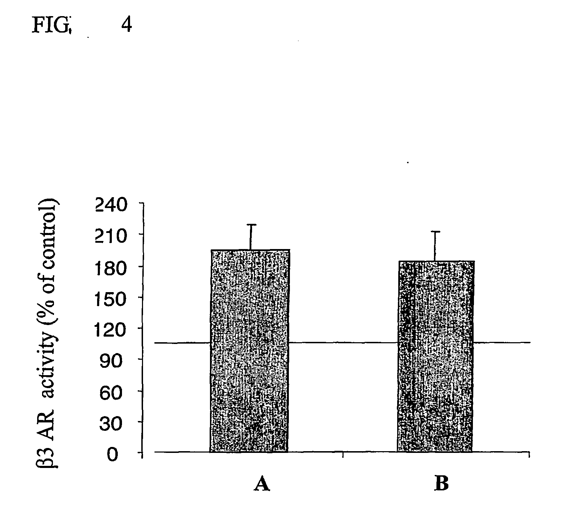

The results are given in figures 2 and 3. Figure 2 shows that the 4 extracts prepared as described in Materials and Methods of example 1 have an effect on β3 adrenoreceptor activity. Figure 3 shows the effects of different doses of the hexane extract of Cyperus rotondus on β3 adrenoreceptor activity. The β3 adrenoreceptor activity of 250 µg/ml of Medicago Sativa (A) and Iris Pallida (B) extracts is illustrated in figure 4.

### Example 4: Dry pet food

A feed mixture is made up of about 58% by weight of com, about 5.5% by weight of corn gluten, about 22% by weight of chicken meal, 2.5% dried chicory, about 10% of capsicum leaves, salts, vitamins and minerals making up the remainder.

The feed mixture is fed into a preconditioner and moistened. The moistened feed is then fed into an extruder-cooker and gelatinised. The gelatinised matrix leaving the extruder is forced through a die and extruded. The extrudate is cut into pieces suitable for feeding to dogs, dried at about 110°C for about 20 minutes, and cooled to form pellets.

This dry dog food is able to control weight of dogs and increase their mobility.

### Example 5: Wet canned pet food with supplement

A mixture is prepared from 73 % of poultry carcass, pig lungs and beef liver (ground), 16 % of wheat flour, 2 % of dyes, vitamins, and inorganic salts. This mixture is emulsified at 12°C and extruded in the form of a pudding, which is then cooked at a temperature of 90°C. It is cooled to 30°C and cut in chunks. 45 % of these chunks are mixed with 55 % of a sauce prepared from 98 % of water, 1 % of dye, and 1 % of guar gum. Tinplate cans are filled and sterilised at 125°C for 40 min.

As a supplement to be mixed with the pet-food before serving, additional packaging (e.g. sachet) contains 25 g of powdered Capsicum leaves to be added to the daily food. The corresponding amount for the pet is about 25 g / day and this can be supplied as a supplement with (e.g. on top of) the can.

This pet food helps to control weight of dogs and to increase their mobility.

## Claims

1. A pet food composition for use in the treatment and/or the prevention of obesity and/or related disorders, which comprises as an active ingredient at least one plant or plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof.

2. A pet food composition for use in controlling the glucose homeostasis of a pet and/or stimulating lipolysis, which comprises a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof.

3. A pet food composition for use in improving activity and/or mobility of a pet, which comprises a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof.

4. A pet food composition according to any of claims 1 to 3, which is in the form of a nutritionally balanced pet food, a dietary supplement or a pharmaceutical composition.

5. A pet food composition according to any of claims 1 to 4, in which the plant or plant extract is present alone or in association with other bioactive molecule such as antioxidants, milk hydrolysates.

6. Use of at least one plant or plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof, as an active ingredient in the manufacture of a pet food composition for the treatment and/or prevention of obesity and/or related disorders.

7. The use of a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof, in the manufacture of a pet food composition for controlling the glucose homeostasis of a pet and/or stimulating lipolysis.

8. The use of a plant or a plant extract having the ability to stimulate energy expenditure and/or fat burning, wherein the plant or plant extract is selected from seeds of *Medicago sativa,* leaves of *Capsicum* species, leaves or tubers of *Cyperus* species or tubers of *Iris Pallida,* or a mixture thereof, in the manufacture of a pet food composition for improving activity and/or mobility of a pet.

9. The use according to any of claims 6 to 8, wherein the composition is in the form of a nutritionally balanced pet food, a dietary supplement or a pharmaceutical composition.

10. The use according to any of claims 6 to 9, in which the plant or plant extract is used alone or in association with other bioactive molecule such as antioxidants, milk hydrolysates.

## Patentansprüche

1. Eine Haustierfutterzusammensetzung für den Einsatz zur Behandlung von und/oder der Vorbeugung gegen Fettleibigkeit und/oder ähnlicher Störungen, die als einen aktiven Bestandteil wenigstens eine Pflanze oder einen Pflanzextrakt umfasst mit der Fähigkeit, Energieaufwendung und/oder Fettverbrennung zu stimulieren, wobei die Pflanze oder der Pflanzextrakt ausgewählt ist aus Keimen von *Medicago sativa,* Blättern von *Capsicum-* Arten, Blättern oder Knollen von Cyperus-Arten oder Knollen von *Iris Pallida,* oder einer Mischung hieraus.

2. Eine Haustierfutterzusammensetzung für den Einsatz zur Kontrolle der Glukosehomöostase eines Haustieres und/oder Stimulation der Lipolyse, die eine Pflanze oder ein Pflanzextrakt mit der Fähigkeit, Energieaufwand und/oder Fettverbrennung zu stimulieren, umfasst, wobei die Pflanze oder der Pflanzextrakt ausgewählt ist aus Keimen von *Medicago sativa,* Blättern von *Capsicum-* Arten, Blättern oder Knollen von *Cyperus*-Arten oder Knollen von *Iris Pallida,* oder einer Mischung hieraus.

3. Eine Haustierfutterzusammensetzung für den Einsatz zur Verbesserung der Aktivität und/oder Mobilität des Haustieres, die eine Pflanze oder ein Pflanzextrakt mit der Fähigkeit, Energieaufwendung und/oder Fettverbrennung zu stimulieren, umfasst, wobei die Pflanze oder der Pflanzextrakt ausgewählt ist aus Keimen von *Medicago sativa,* Blättern von *Capsicum-* Arten, Blättern oder Knollen von *Cyperus-*Arten oder Knollen von *Iris Pallida,* oder einer Mischung hieraus.

4. Eine Haustierfutterzusammensetzung nach einem der Ansprüche 1 bis 3, die in der Form einer ausgewogenen Haustierernährung, eines Nahrungsergänzungsmittels oder einer Arzneimittelzusammensetzung ist.

5. Eine Haustierfutterzusammensetzung nach einem der Ansprüche 1 bis 4, in der die Pflanze oder der Pflanzextrakt allein vorliegt oder in Verbindung mit anderen bioaktiven Molekülen wie Antioxidantien, Milchhydrolysaten.

6. Der Einsatz von wenigstens einer Pflanze oder eines Pflanzextrakts mit der Fähigkeit, Energieaufwand und/oder Fettverbrennung zu stimulieren, wobei die Pflanze oder der Pflanzextrakt ausgewählt ist aus Keimen von *Medicago sativa,* Blättern von *Capsicum-* Arten, Blättern oder Knollen von Cyperus-Arten oder Knollen von *Iris Pallida,* oder einer Mischung hieraus, als aktiven Bestandteil in der Herstellung einer Haustierfutterzusammensetzung für die Behandlung und/oder Vorbeugung von Fettleibigkeit und/oder ähnlichen Störungen.

7. Der Einsatz von wenigstens einer Pflanze oder eines Pflanzextrakts mit der Fähigkeit, Energieaufwand und/oder Fettverbrennung zu stimulieren, wobei die Pflanze oder der Pflanzextrakt ausgewählt ist aus Keimen von *Medicago sativa,* Blättern von *Capsicum-* Arten, Blättern oder Knollen von *Cyperus-Arten* oder Knollen von *Iris Pallida,* oder einer Mischung hieraus, in der Herstellung einer Haustierfutterzusammensetzung für die Kontrolle der Glukosehomöostase eines Haustieres und/oder die Stimulation der Lipolyse.

8. Der Einsatz einer Pflanze oder eines Pflanzextrakts mit der Fähigkeit, Energieaufwand und/oder Fettverbrennung zu stimulieren, wobei die Pflanze oder der Pflanzextrakt ausgewählt ist aus Keimen von *Medicago sativa,* Blättern von *Capsicum-* Arten, Blättern oder Knollen von Cyperus-Arten oder Knollen von *Iris Pallida,* oder einer Mischung hieraus, in der Herstellung einer Haustierfutterzusammensetzung zur Verbesserung der Aktivität und/oder Mobilität eines Haustieres.

9. Der Einsatz nach einem der Ansprüche 6 bis 8, wobei die Zusammensetzung in der Form einer ausgewogenen Haustierernährung, eines Nahrungsergänzungsmittels oder einer Arzneimittelzusammensetzung ist.

10. Der Einsatz nach einem der Ansprüche 6 bis 9, in denen die Pflanze oder der Pflanzextrakt allein verwendet wird oder in Verbindung mit anderen bioaktiven Molekülen wie Antioxidantien, Milchhydrolysaten.

## Revendications

1. Composition alimentaire pour animaux domestiques à utiliser dans le traitement et/ou la prévention de l'obésité et/ou des troubles correspondants, comprenant, comme ingrédient actif, au moins une plante ou un extrait de plante ayant la capacité de stimuler la dépense énergétique et/ou la combustion des graisses, dans laquelle la plante ou l'extrait de plante est choisi(e) parmi des graines de *Medicago sativa,* des feuilles de l'espèce *Capsicum,* des feuilles ou tubercules de l'espèce *Cyperus,* ou des tubercules *d'Iris Pallida,* ou un mélange de ceux-ci.

2. Composition alimentaire pour animaux domestiques, à utiliser dans le contrôle de l'homéostase de glucose d'un animal et/ou la stimulation de la lipolyse, qui comprend une plante ou un extrait de plainte ayant la capacité de stimuler la dépense énergétique et/ou la combustion des graisses, dans laquelle la plante ou l'extrait de plante est choisi(e) parmi des graines de *Medicago sativa,* des feuilles de l'espèce *Capsicum,* des feuilles ou tubercules de l'espèce *Cyperus,* ou des tubercules *d'Iris Pallida,* ou un mélange de ceux-ci.

3. Composition alimentaire pour animaux, à utiliser dans l'amélioration de l'activité et/ou de la mobilité d'un animal domestique, qui comprend une plante ou un extrait de plante ayant la capacité de stimuler la dépense énergétique et/ou la combustion des graisses, dans laquelle la plante ou l'extrait de plante est choisi(e) parmi des graines de *Medicago sativa,* des feuilles de l'espèce *Capsicum,* des feuilles ou tubercules de l'espèce *Cyperus,* ou des tubercules *d'Iris Pallida,* ou un mélange de ceux-ci.

4. Composition alimentaire pour animaux selon l'une quelconque des revendications 1 à 3, qui est sous la forme d'un aliment pour animaux nutritionnellement équilibré, d'un complément alimentaire ou d'une composition pharmaceutique.

5. Composition alimentaire pour animaux selon l'une quelconque des revendications 1 à 4, dans laquelle la plante ou l'extrait de plante est présent(e) seul(e) ou en association avec une autre molécule bioactive comme des antioxydants, des hydrolysats de lait.

6. Utilisation d'au moins une plante ou un extrait de plante ayant la capacité de stimuler la dépense énergétique et/ou la combustion des graisses, dans laquelle la plante ou l'extrait de plante est choisi(e) parmi des graines de *Medicago sativa,* des feuilles de l'espèce *Capsicum,* des feuilles ou tubercules de l'espèce *Cyperus,* ou des tubercules *d'Iris Pallida,* ou un mélange de ceux-ci, comme ingrédient actif dans la fabrication d'une composition alimentaire pour animaux pour le traitement et/ou la prévention de l'obésité et/ou des troubles correspondants.

7. Utilisation d'une plante ou d'un extrait de plante ayant la capacité de stimuler la dépense énergétique et/ou la combustion de graisses, dans laquelle la plante ou l'extrait de plante est choisi parmi des graines de *Medicago sativa,* des feuilles de l'espèce *Capsicum,* des feuilles ou tubercules de l'espèce *Cyperus,* ou des tubercules *d'Iris Pallida,* ou un mélange de ceux-ci, dans la fabrication d'une composition alimentaire pour animaux permettant de contrôler l'homéostase de glucose d'un animal domestique et/ou de stimuler la lipolyse.

8. Utilisation d'une plante ou d'un extrait de plante ayant la capacité de stimuler la dépense énergétique et/ou la combustion des graisses, dans laquelle la plante ou l'extrait de plante est choisi parmi des graines de *Medicago sativa,* des feuilles de l'espèce *Capsicum,* des feuilles ou tubercules de l'espèce *Cyperus,* ou des tubercules *d'Iris Pallida,* ou un mélange de ceux-ci, dans la fabrication d'une composition alimentaire pour animaux permettant d'améliorer l'activité et/ou la mobilité d'un animal.

9. Utilisation selon l'une quelconque des revendications 6 à 8, dans laquelle la composition est sous la forme d'un aliment pour animaux nutritionnellement équilibré, d'un complément alimentaire ou d'une composition pharmaceutique.

10. Utilisation selon l'une quelconque des revendications 6 à 9, dans laquelle la plante ou l'extrait de plante est utilisé(e) seule ou en association avec une autre molécule bioactive comme des antioxydants, des hydrolysats de lait.
